# EUROPÄISCHE PATENTANMELDUNG

(11) **EP 0 885 625 A2**
(43) Veröffentlichungstag der Anmeldung: **23.12.1998**
(21) Anmeldenummer: 98111102.4
(22) Anmeldetag: 17.06.1998
(51) Int. Cl.: A61M 25/10, A01N 1/02

(54) **Katheterset und Verfahren zum Konservieren eines Herzens mittels Koronarperfusion**

(30) Priorität: 19.06.1997 DE 19725959
(71) Anmelder: Eberhard-Karls-Universität Tübingen Universitätsklinikum, 72076 Tübingen (DE)
(72) Erfinder: Scheule, Albertus, 72074 Tübingen (DE)
(74) Vertreter: Witte, Alexander, Dr.-Ing.

(57) **Zusammenfassung**

Ein Katheterset und ein Verfahren dienen zum Konservieren eines Herzens (80) eines kreislauftoten Lebewesens mittels Koronarperfusion. Hierzu umfaßt das Set (10) eine erste Katheteranordnung (11) für die venöse Seite sowie eine zweite Katheteranordnung (12) für die arterielle Seite des Herzens (80). Mittels Blockungsballonen (22, 32, 52) wird das Herz (80) vollständig vom venösen und vom arteriellen Kreislauf abgetrennt, und zwar stromaufwärts der Einmündung der venösen Herzkranzgefäße (86 bis 89) bzw. stromabwärts der arteriellen Herzkranzgefäße (94 bis 97). Auf der arteriellen Seite wird in den Herzkranzgefäß-Kreislauf eine Perfusionslösung unter Druckkontrolle eingebracht und die verbrauchte Perfusionslösung wird auf der venösen Seite wieder abgelassen. Auf diese Weise läßt sich das Herz eines kreislauftoten möglichen Herzspenders über längere Zeiträume konservieren.

## Beschreibung

Die Erfindung betrifft ein Katheterset sowie ein Verfahren zum Konservieren eines Herzens eines kreislauftoten Lebewesens mittels Koronarperfusion.

Es ist bekannt, bei schwer herzkranken Patienten eine Herztransplantation auszuführen. Voraussetzung dafür ist, daß ein geeignetes Spenderherz zur Verfügung steht. Die Zahl der Herztransplantationen geht jedoch gegenwärtig zurück, weil die Organspendebereitschaft der Bevölkerung abnimmt. Die Gründe für diese abnehmende Bereitschaft sind vielfältig und haben einen moraltheologischen, philosophischen oder auch naturwissenschaftlichen Hintergrund. Auch wenn die Mehrzahl der Bevölkerung den Hirntod eines Menschen als endgültigen Todeszeitpunkt betrachtet, ist die Akzeptanz in der Bevölkerung abnehmend. Demzufolge stehen immer weniger Spenderherzen zur Verfügung, so daß Patienten, die auf eine Herztransplantation warten, immer längere Wartezeiten in Kauf nehmen müssen. Bei vielen Patienten wird die Wartezeit so lang, daß sie den Zeitpunkt einer Transplantation nicht mehr erleben.

Nach dem gegenwärtigen Stand der medizinischen Wissenschaft werden ausschließlich Herzen von hirntoten Patienten explantiert und für eine anschließende Transplantation zur Verfügung gestellt. Hierzu wird das Herz unter kreislaufstabilen Verhältnissen im Spender kardioplegiert, explantiert und anschließend kalt gelagert. Während dieser kalten Ischämie wird das Herz vom Spender zum Empfänger transportiert. Die Transportzeit darf nach gegenwärtigem Stand der Wissenschaft maximal fünf Stunden betragen, wobei die Qualität mit zunehmender Ischämiedauer abnimmt.

Entsprechend den gegenwärtigen Verhältnissen geht überdies noch für eine Transplantation wertvolle Zeit verloren, wenn über die Spenderbereitschaft eines möglichen Spenders keine Klarheit besteht, insbesondere deswegen, weil der Spender keinen Organspenderausweis bei sich führt, der eine Organentnahme ermöglichen würde oder weil es nicht möglich ist, mit den Hinterbliebenen des Spenders eine kurzfristige Einigung über eine Organentnahme zu erzielen.

Aus der Zeitschrift "Transplantation Proceedings", Vol. 27, Nr. 5 (Oktober), 1995, Seite 2909 bis 2912, ist ein Katheterset für die Perfusion in situ von Nieren solcher Spender bekannt, deren Herz nicht mehr schlägt. Der bekannte Katheterset umfaßt zwei Blockungsballone mit entsprechender Zuleitung sowie eine Spülleitung, die eine Druckmeßanordnung mindestens näherungsweise am Ort ihres offenen Endes umfaßt.

Der bekannte Katheterset wird über einen Zugang in der Leistenschlagader in diese eingeführt. Er wird mit seinen zwei Blockungsballonen so positioniert, daß der Abschnitt der Aorta, von dem die für die Versorgung der Niere dienenden Arterien abgehen, isoliert wird. Unter Kontrolle des Drucksensors wird nun eine Spülflüssigkeit durch die Spülleitung zugeführt. Zur Ableitung der Spülflüssigkeit wird die Femoralvene eröffnet und über einen entsprechenden Zugang ein Abflußkatheter gelegt.

Eine vergleichbare Anordnung ist auch aus der US 5 505 701 bekannt.

Aus der DE 43 24 637 A1 sind ein Verfahren und ein Bausatz zur Organtransplantation bekannt. Dabei werden für Transplantate im Bauchraum wie z.B. Niere oder Leber jeweils ein arterieller und ein venöser Katheter unter Röntgenkontrolle so plaziert und bei Eintritt des Todes des Patienten kaudal und cranial der Organe liegende Cuffs, also Blockungsballone, elektrisch geblockt und ein Kreislauf über entsprechende Leitungen mittels eines Oxygenerators hergestellt und über Pumpen bevorzugt von der Organentnahme bis zur Transplantation aufrechterhalten. Das hierzu verwendete Katheterset verwendet somit sowohl im arteriellen als auch im venösen Bereich jeweils zwei Blockungsballone. Der venöse Katheter wird über die Vena femoralis eingeführt und in der Vena cava inferior plaziert und mit den Ballonen geblockt. Der arterielle Katheter wird in die Arteria femoralis bis zur Aorta abdominalis eingeführt. Dies geschieht unter Röntgenkontrolle. Der bereits erwähnte Kreislauf wird über Rollenpumpen aufrechterhalten, wobei Blut oder Blutersatzstoffe eingesetzt werden.

In physiologischer Hinsicht bestehen jedoch zwischen Organen des Bauchraums (Niere, Leber) einerseits und dem Herzen andererseits im vorliegenden Zusammenhang erhebliche Unterschiede. Diese Unterschiede betreffen insbesondere die sogenannte Ischämie-Dauer, d.h. die Zeitspanne, die vergeht, bis ein explantiertes Organ für eine anschließende Implantation nicht mehr verwendet werden kann. Dabei unterscheidet man zwischen sogenannter "warmer" und "kalter" Ischämie, je nachdem, ob die Organe nach der Explantation (bzw. der Abtrennung vom Blutkreislauf) im warmen oder im gekühlten Zustand gelagert werden.

Die Organe des Bauchraumes, nämlich Nieren und Lebern, haben insoweit die höchste Ischämie-Toleranz, weil bei Nieren die warme Ischämie bei einer Stunde und die kalte Ischämie bei 36 Stunden liegt. Insoweit sind die Andeutungen in der DE 43 24 637 A1 in Spalte 1, Zeilen 24 bis 30 nicht zutreffend.

Im Gegensatz dazu besitzen Herzen nur eine äußerst niedrige Ischämie-Toleranz. Herzen mit warmer Ischämie werden daher in der Klinik überhaupt nicht transplantiert, sondern nur mit kalter Ischämie, die bei maximal drei bis vier Stunden liegt.

In der Fachwelt existiert daher ein ausgeprägtes Vorurteil dahingehend, daß bei Herzen (anders als bei Nieren oder Lebern) eine Transplantation in warmer Ischämie nicht möglich ist.

Für den hier interessierenden Fall der Konservierung von Organen in einem (noch warmen) Körper eines Spenders ist nämlich von zentraler Wichtigkeit, daß im Falle von Organen des Bauchraumes (Nieren/Lebern) eine Zeitspanne in der Größenordnung von bis zu einer Stunde zur Verfügung steht, während im Falle eines Herzens innerhalb weniger Minuten gehandelt werden muß (tierexperimentelle Ergebnisse). Folglich sind Verfahren, die im Bereich der Transplantation von Organen des Bauchraums einsetzbar sein mögen, nicht auf die Transplantation von Herzen zu übertragen. Dies ist der wesentliche Grund dafür, warum das Fachgebiet der Herztransplantation insoweit eigenständige Wege geht und hinsichtlich der Explantation/Implantation von Organen nicht auf das zurückgegriffen werden kann, was aus der Bauchraum-Chirurgie bekannt ist.

In der DE 43 24 637 ist angegeben, daß die Katheter unter Röntgenkontrolle eingeführt werden. Diese Röntgenkontrolle wird dabei nicht nur benötigt, um den Weg der Katheterspitze vom Einführungsort des Katheters bis hin zur Niere zu verfolgen, was ein relativ zeitaufwendiges räumliches Verschieben des Röntgengerätes bedingt. Es soll darüber hinaus auch die Positionierung der insgesamt vier Ballone relativ zu den abgehenden Versorgungsgefäßen der Nieren überwacht werden. Die ist bei der beschriebenen Konservierung von Nieren besonders wichtig. Werden die Ballonen nämlich in der Arteria femoralis sowie der Vena femoralis nicht exakt positioniert, so werden bei dem beschriebenen Kreislauf nicht nur die Nieren sondern (je nach Lage der Ballone relativ zu den von der Arteria bzw. Vena femoralis abgehenden Gefäßen) auch die Leber bzw. ein größerer Darmbereich mit einbezogen. Dies bedeutet, daß ein erheblicher Zeitaufwand erforderlich ist, um bei dem bekannten Verfahren die Ballone exakt zu positionieren.

Die dafür erforderliche Zeit mag bei einer Konservierung von Organen des Bauchraumes vorhanden sein. Bei einer Konservierung eines Herzens steht diese Zeit keinesfalls zur Verfügung.

Weiterhin ist hinsichtlich des Einsatzes einer Röntgenkontrolle zu berücksichtigen, daß diese nur dann nutzbringend einsetzbar ist, wenn zugleich ein wirksames Kontrastmittel verabreicht wird. Jedwedes Kontrastmittel ist aber gewebeschädlich. Bei den Organen des Bauchraumes besteht auch insoweit eine weitaus höhere Toleranz, verglichen mit einem Herzen. Dies gilt insbesondere dann, wenn das Herz bereits Ischämie-geschädigt ist.

Das bekannte Verfahren kann daher nicht für das Konservieren eines Herzens eingesetzt werden, weil die Verwendung wirksamer Kontrastmittel ausscheidet.

Das bekannte Verfahren zur Vorbereitung einer Organtransplantation lebt von dem Gedanken, daß bereits in situ, d.h. im Körper des Spenders, ein geschlossener Kreislauf eingerichtet wird, um das später zu entnehmende Organ (Niere, Leber) zu versorgen. Innerhalb des geschlossenen Kreislaufes soll Eigenblut oder Fremdblut als Perfusat mittels einer Rollenpumpe gefördert werden.

Beim Fördern von Blut mittels einer Rollenpumpe kommt es jedoch zur Hämolyse. Hämoglobin ist jedoch eine potentiell endotheltoxische Substanz, was zu einer Funktionsminderung des perfundierten Organs führt. Diese potentielle Organschädigung führt zu einer zumindest temporären Funktionsstörung. Während bei Nieren eine solche temporäre Funktionsstörung jedoch mit herkömmlichen Mitteln (Dialyse) verhältnismäßig leicht beherrschbar ist, kommt bei einer temporären Funktionsstörung des Herzens als Ersatzverfahren nur der Einsatz eines Kunstherzens in Betracht. Ein Kunstherz steht jedoch in der Praxis so gut wie nicht zur Verfügung (weltweit derzeit nur wenige Einsätze), während Dialysegeräte in jedem einigermaßen gut ausgerüsteten Krankenhaus vorhanden sind.

Schließlich ist bei dem bekannten Verfahren vorgesehen, einen EKG-gesteuerten geschlossenen Kreislauf einzurichten. Dabei sollen die Ballone nur dann geblockt werden, wenn kein EKG-Signal vorhanden ist. Auf diese Weise soll bei eintretendem Tod durch Herzstillstand des Patienten sogleich der näher beschriebene Kreislauf eingeschaltet werden. Bei einem Wiedereinsetzen des EKG sollen die Ballone sofort wieder entblockt werden, damit im Falle einer Genesung des Patienten wieder die normalen Körperfunktionen aufgenommen werden können. Dabei bleibt unberücksichtigt, daß das Herz auch nach einem Kreislaufstillstand noch weitere EKG-Aktivitäten zeigt. Diese EKG-Aktivitäten, die bei dem bekannten Verfahren zu einem Entblocken der Ballone und zu einem Abschalten des konservierenden Kreislaufs führen würden, können durchaus noch 10 bis 15 Minuten nach Eintritt des Kreislaufstillstandes fortdauern.

Diese Zeitdauer ist jedoch für den Bereich der Organe des Bauchraumes einigermaßen unkritisch, da sie unterhalb der warmen Ischämie liegt. Für den Bereich der Herzkonservierung wäre eine solche Vorgehensweise hingegen fatal, weil das Herzgewebe nach der genannten Zeitdauer von 10 bis 15 Minuten bereits so weit abgestorben wäre, daß das Herz für eine Transplantation nicht mehr in Betracht kommt und eine Konservierung daher sinnlos wäre.

Auch aus diesem Grunde ist das bekannte Verfahren für eine Konservierung von Herzen nicht einsetzbar.

Der Erfindung liegt die Aufgabe zugrunde, einen Katheterset und ein Verfahren der eingangs genannten Art zu schaffen, mit denen es möglich ist, eine neue Gruppe von Herzspendern zu erschließen, so daß die für Transplantationen benötigten Spenderherzen in größerem Maße als bisher zur Verfügung stehen. Insbesondere soll durch eine Konservierung in situ das Herz nach einem Kreislaufstillstand des Patienten im Leichnam konserviert werden, so daß genügend Zeit zur Verfügung steht, um ggf. die erforderliche Einwilligung der Angehörigen des Verstorbenen einzuholen oder ausfindig zu machen, ob der Verstorbene vor seinem Tod einer Organentnahme bereits zugestimmt hat.

Diese Aufgabe wird erfindungsgemäß bei einem Katheterset der eingangs genannten Art dadurch gelöst, daß das Katheterset umfaßt:
a) eine erste Katheteranordnung mit
   - einem ersten Katheter, der einen ersten Blockungsballon enthält,
   - einem zweiten Katheter, der einen zweiten Blokkungsballon enthält, und
   - einem dritte Katheter, der eine erste Spülleitung mit einem ersten offenen Ende enthält;
b) eine zweite Katheteranordnung mit
   - einem vierten Katheter, der einen dritten Blokkungsballon enthält,
   - einem fünften Katheter, der eine zweite Spülleitung mit einem zweiten offenen Ende enthält, und
   - einer Druckmeßanordnung zum Messen des Druckes mindestens näherungsweise am Ort des zweiten offenen Endes;
      wobei die Längen der Katheter so bemessen sind, daß sich
   - bei einer Positionierung der ersten Katheteranordnung in der zum Herzen führenden Vena cava der erste Blockungsballon und der zweite Blockungsballon sich jeweils stromaufwärts beidseits der Einmündung der in den rechten Vorhof des Herzens einmündenden venösen Herzkranzgefäße befinden, wobei das erste offene Ende bei dieser Positionierung zwischen dem ersten und dem zweiten Blockungsballon angeordnet ist, und
   - bei einer Positionierung der zweiten Katheteranordnung in der vom Herzen abgehenden Aorta der dritte Blockungsballon sich im Pars ascendens aortae des Herzens stromabwärts der Einmündung der vom Pars ascendens aortae abgehenden arteriellen Herzkranzgefäße befindet, wobei das zweite offene Ende bei dieser Positionierung stromaufwärts des dritten Blockungsballons angeordnet ist.

Bei einem Verfahren der eingangs genannten Art wird die der Erfindung zugrundeliegende Aufgabe mit den folgenden Schritten gelöst:
a) Legen eines Zuganges in einem venösen Gefäß, das der zum Herzen führenden Vena cava vorgeschaltet ist;
b) Katheterisieren der Vena cava derart, daß ein erster Blockungsballon sich in der Vena cava inferior stromaufwärts der in den rechten Vorhof des Herzens einmündenden venösen Herzkranzgefäße, ein zweiter Blockungsballon sich in der Vena cava superior stromaufwärts der in den rechten Vorhof des Herzens einmündenden venösen Herzkranzgefäße und ein erstes freies Ende einer ersten Spülleitung sich zwischen den Blockungsballonen befinden;
c) Legen eines Zuganges in der vom Herzen aufsteigenden Aorta;
d) Katheterisieren eines aus der Aorta abgehenden Gefäßes derart, daß ein dritter Blockungsballon sich im Pars ascendens aortae stromabwärts der dort abgehenden arteriellen Herzkranzgefäße und ein zweites freies Ende einer zweiten Spülleitung mit einer Druckmeßanordnung sich stromaufwärts des dritten Blockungsballons befinden;
e) Expandieren der Blockungsballone derart, daß die Vena cava inferior, die Vena cava superior sowie der Pars ascendens aortae blockiert werden; und
f) Einbringen einer Herzprotektionslösung durch die zweite Spülleitung und gleichzeitiges Ablassen verbrauchter Perfusionslösung aus der ersten Spülleitung, wobei der Durchsatz der Perfusionslösung in Abhängigkeit von einem Meßsignal der Druckmeßanordnung eingestellt wird.

Die der Erfindung zugrundeliegende Aufgabe wird auf diese Weise vollkommen gelöst.

Erfindungsgemäß wird nämlich das Herz eines kreislauftoten Patienten im Leichnam konserviert, wobei diese Konservierung ohne großen Aufwand über Katheterschleusen von außen bewerkstelligt werden kann, die häufig bei zuvor intensivgepflegten oder unfallversorgten Patienten ohnehin liegen. Der Leichnam selbst braucht in diesem Augenblick nicht weiter beeinträchtigt, insbesondere nicht eröffnet zu werden. Auf diese Weise ist es z.B. möglich, daß die Angehörigen noch in Ruhe von dem Verstorbenen Abschied nehmen können. Es ist somit für alle Beteiligten psychisch erträglicher, zu wissen, daß lediglich das Herz in dem toten Organismus konserviert wird. Die Konservierung des Herzens kann sich dabei auch über längere Zeiträume erstrecken, so daß genügend Zeit zur Verfügung steht, die Voraussetzungen für eine Organspende abzuklären.

Insgesamt ist es daher mit der Erfindung möglich, die Zahl der zur Verfügung stehenden Spenderherzen zu vergrößern, da die Entscheidung über die Organspende nicht unter Zeitdruck erfolgen muß, und zudem der Spender die Zeichen des Todes wie Abkühlung und Totenstarre entwickelt. Dies erleichtert das Abschiednehmen der Angehörigen.

Vergleicht man nun nochmals den Anmeldungsgegenstand mit der Lehre der DE 43 24 637, so ergeben sich wesentliche Unterschiede auch hinsichtlich der bevorzugten Ausführungsform des Kathetersets.

Wie man nämlich ohne weiteres durch Vergleich des Anmeldungsgegenstandes mit dem aus der DE 43 24 637 A1 bekannten Katheterset erkennt, weist die im Rahmen der vorliegenden Anmeldung im arteriellen Bereich eingesetzte zweite Katheteranordnung nur einen einzigen Ballon auf, während der arterielle Katheter des bekannten Sets mit zwei derartigen Ballonen (Cuffs) versehen ist.

Dies liegt daran, daß beim Anmeldungsgegenstand der dritte Blockungsballon im Pars ascendens aortae des Herzens plaziert wird, so daß ein zweiter Blockungsballon nicht mehr erforderlich ist. Die Funktion des zweiten Blockungsballons wird beim Anmeldungsgegenstand nämlich durch die Aortenklappe des Herzens dargestellt.

Auch aus diesem Grunde ist der bekannte Bausatz nicht mit dem angemeldeten Katheterset vergleichbar, weil durch die vorstehend beschriebene Positionierung des dritten Blockungsballons ganz andere Blockierungs- und Strömungsverhältnisse eintreten, als dies bei dem bekannten Bausatz der Fall ist, der vollständig unabhängig von körpereigenen Ventilen arbeitet.

Die Aortenklappe wird beim Anmeldungsgegenstand durch Anlegen eines Überdrucks im arteriellen Zweig vollständig geschlossen gehalten. Es ist folglich für die Funktion des Anmeldungsgegenstandes von elementarer Bedeutung, daß die Funktion der Aortenklappe überwacht wird. Eine solche Funktionsüberwachung kann jedoch nicht durch Röntgenkontrolle bewirkt werden, da mit herkömmlichen Röntgenverfahren der Öffnungs- bzw. Schließzustand der Aortenklappe kaum darstellbar ist, jedenfalls nicht unter dem vorhandenen Zeitdruck.

Für die wesentliche Funktionskontrolle der Aortenklappe ist ein völlig anderes Meßverfahren erforderlich, nämlich die sogenannte Ösophagus-Echocardiographie. Bei diesem Verfahren wird ein Schallaufnehmer in die Speiseröhre (Ösophagus) eingeführt, wobei man sich die Tatsache zunutze macht, daß die Speiseröhre hinter dem Herzen vorbeiläuft. Durch den Schallaufnehmer (Mikrofon) kann in einfacher Weise und schnell die Funktion der Aortenklappe kontrolliert und die Position des Katheters in der aufsteigenden Aorta visualisiert werden.

Bei einer bevorzugten Ausführungsform des erfindungsgemäßen Kathetersets sind bei den Katheteranordnungen jeweils mehrere Katheter baulich vereinigt.

Diese Maßnahme hat den Vorteil, daß die Katheteranordnungen leichter gelegt und mittels transthorakaler, echokardiographischer Kontrolle plaziert werden können.

Insbesondere ist dabei bevorzugt, daß die Druckmeßanordnung einen Drucksensor mit einem Anschlußkabel umfaßt und daß das Anschlußkabel mit dem fünften Katheter baulich vereinigt ist.

Auch diese Maßnahme hat den Vorteil, daß die Druckmeßanordnung in einfacher Weise in einen Katheter integriert werden kann.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: eine schematisierte Darstellung eines Ausführungsbeispiels eines erfindungsgemäßen Kathetersets; und
- Fig. 2: eine schematische Darstellung eines menschlichen Herzens in einem kreislauftoten Patienten, bei dem das erfindungsgemäße Katheterset zum Einsatz gekommen ist.

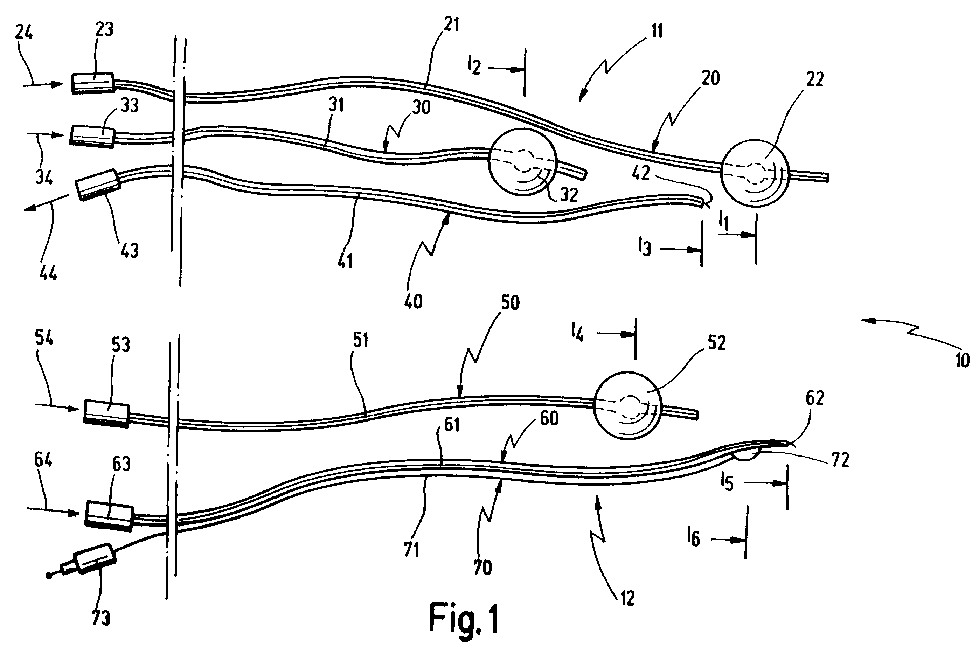

In Fig. 1 ist mit 10 insgesamt ein Katheterset erfindungsgemäßer Art bezeichnet. Der Katheterset 10 umfaßt eine erste, venöse Katheteranordnung 11 sowie eine zweite, arterielle Katheteranordnung 12.

Die erste Katheteranordnung 11 besteht aus drei Kathetern. Ein erster Katheter 20 besteht im wesentlichen aus einem Schlauch 21 und einem ersten Blockungsballon 22. Der Schlauch 21 ist an seinem äußeren Ende mit einem ersten Anschlußstück 23 versehen. Ein erster Pfeil 24 symbolisiert, daß über den Schlauch 21 Flüssigkeit zugeführt werden kann, um den ersten Blockungsballon 22 zu expandieren.

Ein zweiter Katheter 30 ist im wesentlichen baugleich mit dem ersten Katheter 20. Auch er umfaßt einen Schlauch 31 und einen zweiten Blockungsballon 32. An einem zweiten Anschlußstück 33 kann physiologische Flüssigkeit (NaCl) zugeführt werden, wie mit einem zweiten Pfeil 34 angedeutet.

Der dritte Katheter 40 besteht hingegen lediglich aus einem Schlauch 41, der als venöse Ableitung dient. Der Schlauch 41 hat ein offenes Ende 42 und ist an seinem äußeren Ende ebenfalls mit einem dritten Anschlußstück 43 versehen. Ein dritter Pfeil 44 symbolisiert, daß der Schlauch 41 bzw. die Spülleitung zum Ablassen einer Flüssigkeit dient, wie noch erläutert werden wird.

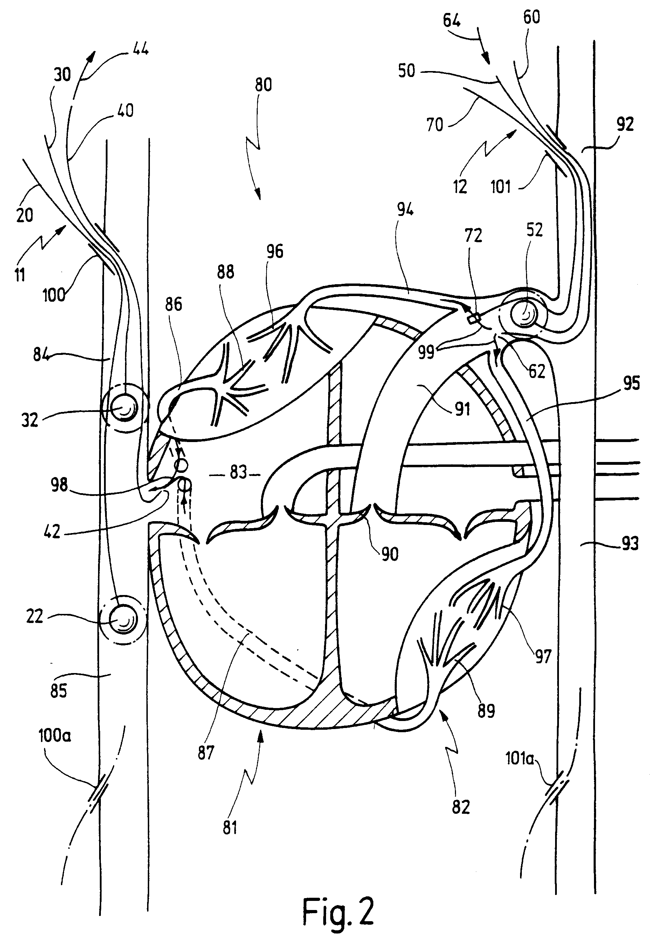

Mit l₁, l₂ und l₃ sind die Längen der Katheter 20, 30, 40 bezeichnet. Die Längen l₁, l₂ und l₃ sind so bemessen, daß sich die Blockungsballone 22, 32 sowie das freie Ende 42 beim Einsatz der ersten Katheteranordnung 11 im venösen Bereich an vor-bestimmten Positionen befinden, wie weiter unten anhand von Fig. 2 noch im einzelnen erläutert werden wird.

Die zweite Katheteranordnung 12 umfaßt ebenfalls drei Katheter.

Ein vierter Katheter 50 umfaßt einen Schlauch 51 und einen dritten Blockungsballon 52. Der Schlauch 51 ist wiederum mit einem vierten Anschlußstück 53 versehen, über das physiologische Flüssigkeit zugeführt werden kann, wie mit einem vierten Pfeil 54 angedeutet.

Ein fünfter Katheter 60 umfaßt im wesentlichen einen Schlauch 61, der als zweite Spülleitung dient. Der Schlauch 61 hat ein offenes Ende 62 und ist am entgegengesetzten Ende mit einem fünften Anschlußstück 63 versehen. Ein fünfter Pfeil 64 am fünften Anschlußstück 63 symbolisiert, daß dem Schlauch 61 bzw. der Spülleitung im Betrieb eine Flüssigkeit zugeführt wird, wie dies weiter unten anhand von Fig. 2 noch erläutert werden wird.

Ein sechster Katheter 70 schließlich hat die Funktion einer Druckmeßanordnung. Der sechste Katheter 70 umfaßt ein Kabel 71 sowie einen Drucksensor 72, der sich in der Nähe des zweiten offenen Endes 62 des fünften Katheters 60 befindet. Der Drucksensor 72 ist über das Kabel 71 mit einem sechsten Anschlußstück 73 verbunden, das in diesem Falle die Gestalt eines elektrischen Steckers hat.

Die Längen der Katheter 50, 60, 70 sind in Fig. 1 mit l₄, l₅ und l₆ bezeichnet. Auch diese Längen l₄, l₅ und l₆ sind nach anatomischen Gesichtspunkten von Herzen bestimmter Größe dimensioniert, wie noch erläutert werden wird.

Fig. 2 zeigt in äußerst schematisierter Weise ein Herz 80 eines bereits kreislauftoten Menschen.

Das Herz 80 umfaßt eine rechte Hälfte 81 sowie eine linke Hälfte 82. Der rechte Vorhof des Herzens 80 ist mit 83 bezeichnet.

Wie aus der Anatomie bekannt, münden in den rechten Vorhof 83 einerseits die Vena cava superior 84 sowie andererseits die Vena cava inferior 85. In der Nähe der Mündung dieser Venen 84, 85 gehen von diesen zwei venöse Herzkranzgefäße ab, nämlich der Sinus coronarius 86 sowie die Vena coronaria dextra 87. Sie dienen zur Versorgung der venösen Herzkranzgefäße 88 für die rechte Herzhälfte 81 bzw. der venösen Herzkranzgefäße 89 für die linke Herzhälfte 82.

Auf der arteriellen Seite steigt von der Aortenklappe 90 des Herzens 80 der Pars ascendens aortae 81 auf. Dieser geht in den Aortenbogen über, aus dem die Kopf- und Armgefäße abgehen. In Fig. 2 ist die Arteria carotis mit 92 und andererseits die nach Abgang der genannten Gefäße aus dem Aortenbogen zur unteren Körperhälfte führende Aorta descendens mit 93 bezeichnet. Vom Pars ascendens aortae 91 geht einerseits die Arteria coronaria dextra 94 und andererseits die Arteria coronaria sinistra 95 ab. Diese Arterien 94, 95 dienen zur Versorgung der arteriellen Herzkranzgefäße 96 für die rechte Herzhälfte 81 bzw. der arteriellen Herzkranzgefäße 97 für die linke Herzhälfte 82.

Mit einem Pfeil 98 ist auf der venösen Seite angedeutet, daß das verbrauchte Blut aus dem Herzkranzkreislauf über die erwähnten Venen 86, 87 in den rechten Vorhof 83 einströmt, während das frische, mit Sauerstoff versorgte Blut in Richtung der Pfeile 99 aus dem Pars ascendens aortae 91 in die erwähnten Arterien 94, 95 abströmt, um den Herzkranzkreislauf eingangsseitig zu versorgen.

Mit dem erfindungsgemäßen Katheterset ist es nun möglich, das Herz 80 vollständig vom venösen und vom arteriellen Kreislauf des Körpers abzutrennen und nur noch den kleinen Herzkranzkreislauf mittels Perfusion einer geeigneten Protektionslösung zu versorgen.

Zu diesem Zweck wird die erste Katheteranordnung 11, z.B. über die Vena jugularis, in den Venen 84, 85 so positioniert, daß sich der erste Blockungsballon 22 in der Vena cava inferior 85 stromaufwärts der Einmündung der vena coronaria dextra 87 befindet, während der zweite Blockungsballon 32 in der Vena cava superior 84 stromaufwärts der Einmündung des Sinus coronarius 86 positioniert ist. Das offene Ende 42 des dritten Katheters 40, d.h. der Spülleitung 41, befindet sich zwischen den Blockungsballonen 22, 32 und ragt vorzugsweise bis in den rechten Vorhof 83 hinein.

Die zweite Katheteranordnung 12 ist demgegenüber, z.B. über die Arteria subclavia dextra, im arteriellen Teil positioniert. Der dritte Blockungsballon 52 befindet sich im Pars ascendens aortae 91, und zwar stromabwärts des Abganges der Aorten 94, 95. Das offene Ende 62 des fünften Katheters 60, d.h. der Spülleitung 61 befindet sich zwischen den Abgängen der Herzkranzgefäße und dem dritten Blockungsballon 52, und der Drucksensor 72 ist in der Nähe des offenen Endes 62 angeordnet bzw. mit diesem baulich vereint, wie in Fig. 1 dargestellt.

Zum Legen der Katheteranordnungen 11 und 12 muß zunächst ein Zugang gelegt werden, wobei bevorzugt die Halsvene, d.h. die Vena cava superior 84 bzw. die Halsschlagader, d.h. die Arteria carotis 92 eröffnet und mit einem Zugang 100 bzw. 101 versehen werden. Alternativ ist es aber auch möglich, die Leistenvene bzw. die Leistenschlagader zu eröffnen, wie mit Zugängen 100a bzw. 101a in Fig. 2 angedeutet.

Sobald die Katheteranordnungen 11, 12 so liegen, wie vorstehend beschrieben und in Fig. 2 dargestellt, werden die Blockungsballone 22, 32 und 52 expandiert, mit der Folge, daß die Vena cava superior 84, die Vena cava inferior 85 sowie der Pars ascendens aortae 91 vollständig blockiert werden. Das Herz 80 ist damit vollständig vom venösen und vom arteriellen Kreislauf abgetrennt. Die Lage der Katheteranordnungen 11, 12 kann leicht nicht-invasiv mit Hilfe der Echokardiographie transthorakal bestimmt werden.

Über den fünften Katheter 60, d.h. die zweite Spülleitung 61 wird nun eine Perfusionslösung in den Pars ascendens aortae 91 eingeleitet. Gleichzeitig wird mittels des Drucksensors 72 der dort herrschende Druck überwacht. Der Durchsatz der Perfusionslösung wird dabei so eingestellt, daß ein bestimmter Druck-Grenzwert nicht überschritten wird.

Aufgrund des Vorhandenseins des expandierten dritten Blockungsballons 52 fließt die Perfusionslösung bei geschlossener Aortenklappe 90 nun ausschließlich über die arteriellen Herzkranzgefäße 94, 95, 96, 97 in Richtung der Pfeile 99 und gelangt dann in verbrauchtem Zustand über die venösen Herzkranzgefäße 86, 87, 88, 89 in Richtung der Pfeile 98 in den rechten Vorhof 83. Vom rechten Vorhof 83 fließt die verbrauchte Perfusionslösung nun durch die erste Spülleitung 41 des dritten Katheters 40 ab.

Mittels des unter Durchleuchtungskontrolle gelegten Kathetersets 10 ist somit ein vollständiger Kreislauf für die Herzkranzgefäße 86 bis 89, 94 bis 97 installiert, so daß sich das Herz 80 in dem bereits kreislauftoten Körper eines Verstorbenen für einen längeren Zeitraum konservieren läßt. Experimentelle Studien haben bereits ergeben, daß die Konservierungszeit im Körper mindestens einige Stunden betragen kann, doch werden wohl ein bis zwei Stunden im Körper ausreichen.

Sobald darüber entschieden ist, daß eine Explantation des Herzens 80 stattfinden kann, wird dieses revitalisiert.

## Patentansprüche

1. Katheterset zum Konservieren eines Herzens (80) mittels Koronarperfusion, wobei das Katheterset (10) umfaßt:
a) eine erste Katheteranordnung (11) mit
- einem ersten Katheter (20), der einen ersten Blockungsballon (22) enthält,
- einem zweiten Katheter (30), der einen zweiten Blockungsballon (32) enthält, und
- einem dritten Katheter (40), der eine erste Spülleitung (41) mit einem ersten offenen Ende (42) enthält;
b) eine zweite Katheteranordnung (12) mit
- einem vierten Katheter (50), der einen dritten Blockungsballon (52) enthält,
- einem fünften Katheter (60), der eine zweite Spülleitung (61) mit einem zweiten offenen Ende (62) enthält, und
- einer Druckmeßanordnung (71, 72) zum Messen des Drucks mindestens näherungsweise am Ort des zweiten offenen Endes (62);
wobei die Längen (l₁, l₂, l₃, l₄, l₅) der Katheter (20, 30, 40, 50, 60) so bemessen sind, daß sich
- bei einer Positionierung der ersten Katheteranordnung (11) in der zum Herzen (80) führenden Vena cava (84, 85) der erste Blockungsballon (22) und der zweite Blockungsballon (32) sich stromaufwärts der Einmündung der in die Vena cava (84, 85) einmündenden venösen Herzkranzgefäße (86, 87, 88, 89) befinden, wobei das erste offene Ende (42) bei dieser Positionierung zwischen dem ersten und dem zweiten Blockungsballon (22, 32) angeordnet ist, und
- bei einer Positionierung der zweiten Katheteranordnung (12) in der vom Herzen (80) abgehenden Aorta (92, 93) der dritte Blockungsballon (52) sich im Pars ascendens aortae (91) des Herzens (80) stromabwärts der Einmündung der vom Pars ascendens aortae (91) abgehenden arteriellen Herzkranzgefäße (94, 95, 96, 97) befindet, wobei das zweite offene Ende (62) bei dieser Positionierung stromaufwärts des dritten Blockungsballons (52) angeordnet ist.

2. Katheterset nach Anspruch 1, dadurch gekennzeichnet, daß bei den Katheteranordnungen (11, 12) jeweils mehrere Katheter baulich vereinigt sind.

3. Katheterset nach Anspruch 1 oder 2, dadurch gekennzeichnet, daß die Druckmeßanordnung einen Drucksensor (72) mit einem Anschlußkabel (71) umfaßt und daß das Anschlußkabel (71) mit dem fünften Katheter (60) baulich vereinigt ist.

4. Verfahren zum Konservieren eines Herzens (80) eines kreislauftoten Lebewesens mittels Koronarperfusion, mit den Schritten:
a) Legen eines Zuganges (100; 100a) in der zum Herzen (80) führenden Vena cava (84, 85);
b) Katheterisieren der Vena Cava (84, 85) derart, daß ein erster Blockungsballon (22) sich in der Vena cava inferior (85) stromaufwärts der dort einmündenden venösen Herzkranzgefäße (87, 89), ein zweiter Blockungsballon (32) sich in der Vena cava superior (84) stromaufwärts der dort einmündenden venösen Herzkranzgefäße (86, 88) und ein erstes freies Ende (42) einer ersten Spülleitung (40) sich zwischen den Blockungsballonen (22, 32) befinden;
c) Legen eines Zuganges (101; 101a) in der vom Herzen (80) abgehenden Aorta (92, 93);
d) Katheterisieren der Aorta (101, 101a) derart, daß ein dritter Blockungsballon (52) sich im Pars ascendens aortae (91) stromabwärts der dort abgehenden arteriellen Herzkranzgefäße (94, 95, 96, 97) und ein zweites freies Ende (62) einer zweiten Spülleitung (61) mit einer Druckmeßanordnung (71, 72) sich stromaufwärts des dritten Blockungsballons (52) befinden;
e) Expandieren der Blockungsballone (22, 32, 52) derart, daß die Vena cava inferior (85), die Vena cava superior (84) sowie der Pars ascendens aortae (91) blockiert werden;
f) Einbringen (64) einer frischen Perfusionslösung durch die zweite Spülleitung (60) und gleichzeitiges Ablassen (44) verbrauchter Perfusionslösung aus der ersten Spülleitung (40), wobei der Durchsatz der Perfusionslösung in Abhängigkeit von einem Meßsignal der Druckmeßanordnung (71, 72) eingestellt wird.
